(19) Europäisches Patentamt
European Patent Office
Office européen des brevets

(11) Veröffentlichungsnummer: **0 354 319**
**A1**

# (12) EUROPÄISCHE PATENTANMELDUNG

(21) Anmeldenummer: **89110645.2**

(22) Anmeldetag: **13.06.89**

(51) Int. Cl.[4]: **C07D 233/54 , C08G 59/40**

Patentanspruch für folgenden Vertragsstaat: ES

(30) Priorität: **07.07.88 DE 3822959**

(43) Veröffentlichungstag der Anmeldung:
**14.02.90 Patentblatt 90/07**

(84) Benannte Vertragsstaaten:
**AT BE CH DE ES FR GB IT LI NL SE**

(71) Anmelder: **SCHERING AKTIENGESELLSCHAFT**
**Berlin und Bergkamen**
**Waldstrasse 14 Postfach 15 40**
**D-4709 Bergkamen(DE)**

(72) Erfinder: **Burba, Christian, Dr. Dipl.-Chem.**
**Gerhart-Hauptmann-Strasse 9**
**D-41715 Ascheberg(DE)**
Erfinder: **Mrotzek, Werner, Dr., Dipl.-Ing.**
**Dresdener Strasse 24**
**D-4600 Dortmund 1(DE)**

(54) **Substituierte 3-(N-Imidazolyl)-propion-säurehydrazide, ihre Verwendung als Härtungsmittel in Epoxidharzzusammensetzungen, sie enthaltende härtbare Epoxidharzzusammensetzungen und Epoxidharzformkörper.**

(57) Gegenstand der Erfindung sind substituierte 3-(N-Imidazolyl)-propionsäurehydrazide, Ihre Verwendung als Härtungsmittel in Epoxidharzzusammensetzungen, sie enthaltende härtbare Epoxidharzzusammensetzungen bestehend aus a) einem Epoxidharz und gegebenenfalls aus b) Dicyandiamid und gegebenenfalls c) üblichen stickstoffhaltigen heterocyclischen Aminverbindungen zur Herstellung von Formkörpern.

**EP 0 354 319 A1**

# Substituierte 3-(N-Imidazolyl)-propionsäurehydrazide, ihre Verwendung als Härtungsmittel in Epoxidharzzusammensetzungen, sie enthaltende härtbare Epoxidharzzusammensetzungen und Epoxidharzformkörper

Gegenstand der Erfindung sind substituierte 3-(N-Imidazolyl)-propionsäurehydrazide, ihre Verwendung als Härtungsmittel in Epoxidharzzusammensetzungen, sie enthaltende härtbare Epoxidharzzusammensetzungen bestehend aus a) einem Epoxidharz und gegebenenfalls aus b) Dicyandiamid und gegebenenfalls c) üblichen stickstoffhaltigen heterocyclischen Aminverbindungen zur Herstellung von Formkörpern.

Für die Herstellung von Verbundwerkstoffen werden heute im Prinzip zwei Verfahren angewandt: Das Naß-in-Naß-Verfahren, bei welchem die Verstärkungsmaterialien mit der härtbaren Mischung imprägniert, in feuchtem Zustand unter Formgebung aufeinandergelegt und in der Hitze in einer Stufe bis zum duromeren Endzustand ausgehärtet werden.

Beim Zweistufenverfahren werden aus den Verstärkungsmaterialien und der härtbaren Mischung zunächst sogenannte Prepregs hergestellt, die dann in einem zeitlich getrennten 2. Verfahrensschritt zu Fertigteilen weiterverarbeitet werden. Dabei ist verfahrenstechnisch noch einmal zwischen der Verwendung lösungsmittelhaltiger und lösungsmittelfreier Arbeitsweise zu unterscheiden.

Die Herstellung der Prepregs erfolgt normalerweise in einem kontinuierlichen Prozeß, in dem die Verstärkungsmaterialien entweder durch ein Imprägnierbad des einzusetzenden Harz-Härter-Gemisches geleitet werden oder das Imprägniermittel wird erst unmittelbar vor seinem Auftrag auf das Trägermaterial vermischt und mittels einer besonderen Vorrichtung aufgerakelt. Die Steuerung der auf einer bestimmten Trägermaterialbahn aufzubringenden Imprägniermittelmenge erfolgt dabei außer über die Viskosität des Imprägniermittels über nachgeschaltete Abquetschwalzen.

Bei lösungsmittelhaltigen Systemen wird nach dem Imprägnierprozeß durch Wärmezufuhr das in der Imprägnierlösung enthaltene Lösungsmittel verdampft und gleichzeitig das Harzsystem vom A- in den B-Zustand überführt. Aus dem mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösungsmittel der Imprägniermischung vollständig entzogen wird, andererseits aber der für die Prepreg-Härtung im 2. Verfahrensschritt notwendige latente Härter noch nicht anspringt, um ein ungewolltes Ausreagieren der imprägnierten Verstärkungsmaterialien zu verhindern.

Bei lösungsmittelfreien Systemen erfolgt abhängig von der chemischen Zusammensetzung des Harzsystems nach der Imprägnierung entweder ebenfalls eine kurze Wärmebehandlung des Materials, um das Imprägniermittel in den B-Zustand zu überführen, oder die Verstärkungsstoffe werden unmittelbar nach der Imprägnierung unter Verzicht auf eine gesonderte Wärmebehandlung beidseitig mit Trennfolien kaschiert und einer systemadäquaten Zwischenlagerung zugeführt. Im Verlaufe dieser Zwischenlagerung tritt entweder ein allmählicher Übergang des Harzsystems in den B-Zustand ein, oder das Imprägniermittel wird unter weitgehendem Verzicht auf chemische Veränderungen allein durch physikalische Effekte auf den Trägermaterialien fixiert.

Die so erhaltenen Prepregs lassen sich als Rollen zwischenlagern und transportieren, ehe sie dem jeweiligen Anwendungsfall entsprechend zurechtgeschnitten und in Bauteildicke übereinander geschichtet werden. Durch gleichzeitige Druck- und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb Raumtemperatur von der Praxis immer weniger akzeptiert.

Von Bedeutung ist weiterhin, daß je nach Herstellungsverfahren der Prepregs die Materialviskosität der gebrauchsfertigen härtbaren Mischung über einen möglichst langen Zeitraum weitgehend unverändert bleibt. Speziell bei Verwendung eines großvolumigen Tränkbades ist dies für die Erzielung eines konstanten Harzauftrages und eines gleichbleibenden B-Zustandes erforderlich, da zum einen die Bedingungen der Produktion nicht laufend den sich ändernden Verhältnissen in der härtbaren Mischung angepaßt werden können und zum anderen dadurch auch die physikalischen Eigenschaften des ausgehärteten Endproduktes negativ beeinflußt werden.

Angestrebt wird in der Praxis eine härtbare Mischung, welche im Imprägnierbad auch über längere Zeit viskositätsmäßig konstant bleibt, bei geringen Temperaturen in kurzer Zeit zum B-Zustand reagiert und dann als Prepreg bei Raumtemperatur ohne chemische Veränderungen lange gelagert werden kann.

Unabhängig von der Prepregherstellung soll ihre Aushärtung bei möglichst tiefen Temperaturen innerhalb kurzer Zeit erfolgen, das Temperaturmaximum der exothermen Reaktion auch bei größeren Schichtdicken auf einem niedrigen Niveau verbleiben und das physikalische Eigenschaftsniveau der Endprodukte den Erfordernissen der Praxis angepaßt sein, d. h. insbesondere, daß die von der Matrix bestimmten Übergangstemperaturen oberhalb von 140 °C liegen. Diese Forderungen hinsichtlich des Härtungsverhaltens und Eigenschaftsviveaus gelten in gleicher Weise auch für die im Naß-in-Naß-Verfahren zu verarbeitenden Epoxidharzsysteme.

Das in härtbaren Mischungen auf Basis von Epoxidharzen seit langem als Härter verwendete Dicyandiamid wird zur Erzielung der angestrebten Eigenschaften in der Regel mit Co-Härtungsmitteln und/oder Beschleunigern kombiniert. Aus der Literatur sind auf diesem Gebiet daher eine Vielzahl von Vorschlägen bekannt geworden.

Die Mitverwendung von tertiären Aminen wie Benzyldimethylamin, tertiär/sekundären Aminen wie 2-Methyl-imidazol, 2-Ethyl-4-methyl-imidazol, tertiär/primären Aminen wie 1-Aminoethyl-imidazol allein oder in Mischungen brachte zwar graduelle Verbesserungen, ohne jedoch gravierende Mängel beseitigen zu können.

Aus der Literatur ist weiterhin die Verwendung von Dicarbonsäuredihydraziden neben Dicyandiamid als latentes Härtungsmittel für Epoxidharze vorgeschlagen worden.

Diese Hydrazide weisen jedoch den Nachteil auf, daß die Anspringtemperaturen zum Teil recht hoch liegen und daß eine vollständige Härtung erst durch Anwendung von Temperaturen um 150 °C und höher über einen langen Zeitraum erreicht werden kann.

Aus der EP-A-106 635 sind Hydrazide bekannt, welche durch Addition von Acrylsäureestern an geradkettige aliphatische polyfunktionelle Amine und anschließende Umsetzung mit Hydrazinhydrat zu den entsprechenden polyfunktionellen Hydraziden hergestellt werden. Die Forderungen der Praxis nach Anspringtemperaturen um 100 °C bei gleichzeitig mög lichst geringen Exothermiemaxima, kurzen Aushärtungszeiten und Glasübergangstemperaturen $\geq$ 140 °C werden auch von diesen Produkten nicht erfüllt.

Aufgabe der vorliegenden Erfindung war es, unter Vermeidung der Nachteile des Standes der Technik härtbare Mischungen auf Basis von Epoxidverbindungen und latenten Härtungsmitteln zu finden, welche bei relativ niedrigen Temperaturen innerhalb kurzer Zeiten ohne hohe exotherme Temperaturspitzen zum duromeren Endzustand aushärten, eine den Anforderungen der Praxis entsprechende thermische Beständigkeit besitzen und welche in Prepregs bei Raumtemperatur über eine ausreichende Lagerstabilität verfügen.

Diese Aufgabe wird gelöst durch Verwendung eines neuen Härtungsmittels, gegebenenfalls unter Mitverwendung üblicher latenter Härtungsmittel.

Gegenstand der Erfindung sind daher Verbindungen der allgemeinen Formel (I)

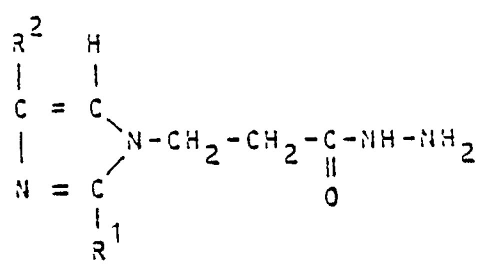

(I)

in welcher $R^1$ und $R^2$ unabhängig voneinander die Bedeutung H, $-CH_3$, $-C_2H_5$, Phenyl haben können.

Ein weiterer Gegenstand der Erfindung ist die Verwendung von Verbindungen der allgemeinen Formel (I),

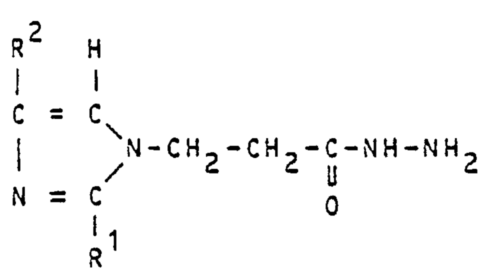

(I)

in welcher $R^1$ und $R^2$ unabhängig voneinander die Bedeutung H, $-CH_3$, $-C_2H_5$, Phenyl haben können, gegebenenfalls unter Mitverwendung üblicher stickstoffhaltiger heterocyclischer Aminverbindungen als Härtungsmittel für Epoxidharze.

Ein weiterer Gegenstand der Erfindung sind härtbare Epoxidharz-Zusammensetzungen enthaltend

a) ein Epoxidharz mit durchschnittlich mehr als einer Epoxidgruppe pro Molekül und gegebenenfalls

b)Dicyandiamid und gegebenenfalls

c) Lösungsmittel, Füllstoffe, Verstärkungs- oder Einlagematerialien, Pigmente, Hilfsstoffe und

d)Verbindungen der allgemeinen Formel (I)

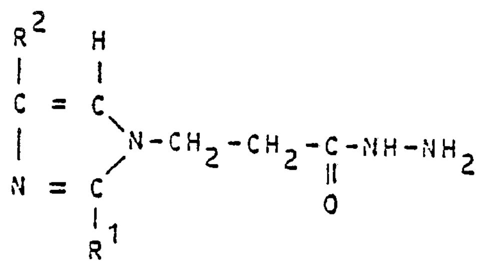

(I)

in welcher $R^1$ und $R^2$ unabhängig voneinander die Bedeutung H, $-CH_3$, $-C_2H_5$, Phenyl haben können und gegebenenfalls

e) übliche stickstoffhaltige heterocyclische Aminverbindungen.

Ein weiterer Gegenstand der Erfindung sind härtbare Epoxidharz-Zusammensetzungen, worin die Verstärkungs- oder Einlagematerialien bei Raumtemperatur mit dem Bindemittel bestehend aus

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

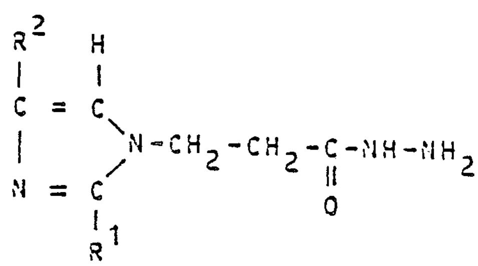

(I)

in welcher $R^1$ und $R^2$ unabhängig voneinander die Bedeutung H, $-CH_3$, $-C_2H_5$, Phenyl haben können und gegebenenfalls

e) üblichen stickstoffhaltigen heterocyclischen Aminverbindungen getränkt und gegebenenfalls bei erhöhter Temperatur in den halbfesten aber noch schmelzbaren Zustand (B-Zustand) überführt werden.

Ein weiterer Gegenstand der Erfindung sind Epoxidharzformkörper, welche dadurch gekennzeichnet sind, daß in erster Stufe die Verstärkungs- oder Einlagemateralien bei Raumtemperatur mit dem Bindemittel, bestehend aus

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

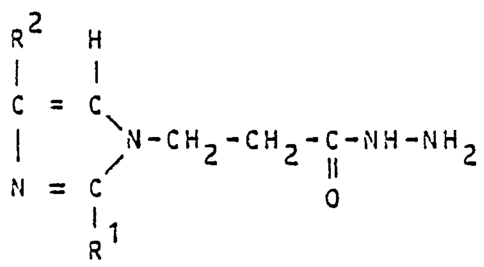

(I)

in welcher $R^1$ und $R^2$ unabhängig voneinander die Bedeutung H, $-CH_3$, $-C_2H_5$, Phenyl haben können und gegebenenfalls

e) üblichen stickstoffhaltigen heterocyclischen Aminverbindungen getränkt und gegebenenfalls in den halbfesten aber noch schmelzbaren Zustand (B-Zustand) überführt werden und in zweiter Stufe die feuchten Laminate oder Prepregs allein und unter Formgebung oder zwischen zu verklebende Substrate gebracht, unter Anwendung von Druck bei erhöhter Temperatur ausgehärtet werden.

Die weiteren Gegenstände der Erfindung sind durch die Ansprüche gekennzeichnet.

Die erfindungsgemäß mitverwendeten Epoxidharze sind Glycidylester und -ether mit zwei oder mehr Epoxygruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 - 0,6, insbesondere die bei Raumtemperatur flüssigen Verbindungen mit Epoxidwerten um 0,45 bis 0,55. Daneben haben sich auch die Glycidylether auf Basis von Bisphenol F und die Novolake als vorteilhaft erwiesen.

Das als Härtungsmittel gebenenfalls mitverwendete Dicyandiamid ist ein handelsübliches Produkt und ist unter den bekannten Handelsnamen erhältlich. Die Menge an Dicyandiamid liegt in Abhängigkeit von der eingesetzten Epoxidverbindung im Bereich von 2 bis 10, bei den erfindungsgemäß bevorzugten flüssigen Glycidylethern auf Basis von Bisphenol A im Bereich von 5 bis 10 Gewichtsteilen, bezogen auf 100 Gewichtsteile Diglycidylether.

Die erfindungsgemäß verwendeten Härtungsmittel bzw. Härtungsbeschleuniger sind Verbindungen der allgemeinen Formel (I),

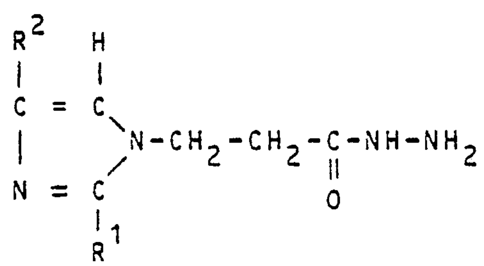

(I)

Die Menge an Härtungsmitteln bzw. Härtungsbeschleunigern kann innerhalb weiter Grenzen variiert werden. Sie wird bestimmt durch den beabsichtigten Anwendungszweck und die gegebenenfalls dadurch vorgegebenen Härtungsbedingungen. Erfindungsgemäß werden Mengen im Bereich von 0,1 bis 40, vorzugsweise 1 bis 10 Gewichtsteilen, bezogen auf 100 Gewichtsteile Epoxidverbindung, angewandt.

Als erfindungsgemäß mitzuverwendende übliche stickstoffhaltige heterocyclische Aminverbindungen können N-Alkylimidazole wie N-Methyl, N-Ethylimidazol und/oder Imidazolinverbindungen der allgemeinen Formel II

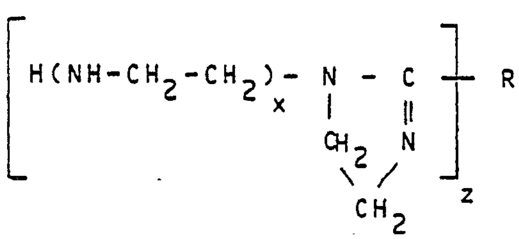

(II)

worin R ein gegebenenfalls verzweigter Alkyl- oder Alkylenrest mit < 10 C-Atomen, insbesondere $-CH_3$,

-CH(OH-$CH_3$, -$(CHR')_y$, worin R′ = H oder -$CH_3$bedeuten und x = 1, 2, 3 und y = 4 - 8 und z gleich der Wertigkeit von R ist, mitverwendet werden, insbesondere solche, in denen x = 1, z = 1 und R = -$CH_3$oder -$CH_2$-$CH_3$ ist.

Zur Modifizierung der Eigenschaften des Endprodukts können neben anderen EP-Harzen auch Modifizierungs- oder Hilfsstoffe mitverwendet werden wie Phenolharze, Melaminharze, Silikonharze, anorganische und organische Füllstoffe wie Quarzmehle, Titandioxid, Ruß, Silikon- oder Butadienkautschuk.

Zur Einstellung der gewünschten Viskosität können unterschiedlich viskose Harze oder Verdünnungsmittel mitverwendet werden, aber auch die üblichen Lösungsmittel wie Dimethylformamid, Aceton, Methylethylketon, Methylglykol, Propylenglykolmonomethylether oder deren Mischungen eingesetzt werden.

Zur Herstellung der Prepregs werden organische und anorganische Fasern, Vliese und Gewebe auf Basis von Aramid, Kohlenstoff oder Cellulose, Metallen wir Bor, Stahl, usw. Keramik, insbesondere aber Glas, mitverwendet.

Die Herstellung der lösungsmittelhaltigen Prepregs geschieht nach der an sich bekannten Methode, bei der die Trägermaterialien in einem Imprägnierbad mit der reaktiven Harzmischung getränkt und nach dem Abquetschen der überschüssigen Harzmenge kontinuierlich unter Energiezufuhr (meist Wärme), bei gleichzeitigem Entzug des Lösungsmittels, vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen unter gleichzeitiger Wärmezufuhr ein hochvernetztes Werkstück entsteht.

Die erfindungsgemäßen Härtungsmittel können außerdem mit Erfolg in lösungsmittelfreien Systemen auf Basis von Epoxidharzen und gegebenenfalls Dicyandiamid verwendet werden. Beispiele sind lagerstabile lösungsmittelfreie Prepregs mit und ohne Dicyandiamid, Naßlaminate, heißhärtende Einkomponentenklebstoffe für die Verklebung von Karosserieteilen in der Automobilindustrie (Bördelnahtkleber) und Epoxidharzgießlinge, Epoxidharz-Beschichtungsmittel oder -wickelkörper.

## Beispiele

### I. Herstellung eines erfindungsgemäßen Härters bzw. Beschleunigers aus 2-Ethyl-4-methylimidazol (EMI-2,4), Acrylsäure-methylester und Hydrazin

55 g EMI-2,4 (0,5 Mol) werden in 55 ml Methanol unter Rühren bei 50 °C vorgelegt. Zu der Lösung tropft man langsam 43 g Acrylsäuremethylester (0,5 Mol) und läßt 1 h bei 50 °C nachreagieren. Dieses Reaktionsgemisch wird langsam zu einer, auf 70 °C vorgewärmten Lösung von 31,3 g Hydrazinhydrat 80 %ig (0,5 Mol Hydrazin) in 50 ml Methanol zugetropft. Man läßt 3 h bei 70 °C nachreagieren und kontrolliert die Vollständigkeit der Hydrazid-Bildung im IR-Spektrum (Verschwinden der Esterbande).

Nach Abziehen des Methanols im Rotavapor erhält man eine viskose, gelbliche, zur Kristallation neigende Flüssigkeit, die ohne weitere Reinigung als Beschleuniger eingesetzt werden kann.

### I a. Herstellung einer Prepreg-Reaktionsmischung aus I unter Mitverwendung von Dicyandiamid

Die bei Raumtemperatur hergestellte Lösung von
16 g Dicyandiamid und
3 g Reaktionsprodukt I in
101 g Dimethylformamid p. a.
wird mit 60 phr mit einem Epoxidharz (Epoxid-Äquivalentgewicht ca. 190) abgemischt und zur Prepreg-Herstellung eingesetzt.

Die Herstellung der Prepregs im Labormaßstab erfolgt durch Aufstreichen der Imprägnierlösung auf ein ca. 0,1 $m^2$ großes Glasfilamentgewebe in Atlasbindung (296 g/$m^2$), welches nach der Imprägnierung 5 min bei 100 °C im Umluftofen thermisch behandelt wird. Nach dem Abdampfen des Lösungsmittels und dem Übergang des Harzsystems vom A- in den B-Zustand resultieren flexible, leicht klebende Prepregs, die auch nach mehrwöchiger Lagerung zwischen Polyethylen-Folien bei Raumtemperatur noch zu hochfesten Formteilen weiterverarbeitbar sind.

Zur Ermittlung der Übergangstemperatur des ausreagierten Harz-Härter-Gemisches wurden jeweils die wie oben beschrieben hergestellten Prepreglagen im Heißpreßverfahren bei 120 °C und 0,1 bar 30 min

lang verpreßt. Das derart ausgehärtete Endprodukt zeigt keinerlei Mängel bezüglich der Haftung der einzelnen Prepreglagen aufeinander und besitzt nach Tabelle 1 eine Übergangstemperatur von 148 °C.

Die ebenfalls noch in dieser Tabelle enthaltenen Viskositäten der Imprägnierlösungen wurden mittels eines Kegel-Platte-Rheometers der Firma Epprecht Instruments ermittelt, wobei die Lösungen zwischen den einzelnen Messungen in einem verschlossenen Behältnis bei Raumtemperatur gelagert wurden.

Die in dieser Tabelle zusätzlich noch enthaltenen Anspringtemperaturen der Imprägnierlösungen und die Temperaturen bei Überschreiten des Exothermiemaximums (Peak-Temperatur) wurden mit einem DSC-Gerät TA 3000 mit Meßzelle DSC 30 der Firma Mettler bei einer Starttemperatur von 20 °C und Aufheizraten von 10 bzw. 20 °C/min ermittelt.

I b Herstellung einer Prepreg-Reaktionsmischung aus I unter Mitverwendung von Dicyandiamid

Die bei Raumtemperatur hergestellte Lösung von
16 g Dicyandiamid und
6 Reaktionsprodukt I in
98 g Dimethylformamid p. a.
wird mit 60 phr mit einem Epoxidharz (Epoxid-Äquivalentgewicht ca. 190) abgemischt, um daraus entsprechend der unter Beispiel I a beschriebenen Methode bei Raumtemperatur lagerfähige Prepregs und hochfeste Endprodukte herzustellen. Die für dieses Beispiel charakteristischen Merkmale sind wie für Beispiel I a in Tabelle 1 wiedergegeben.

II Herstellung eines nicht erfindungsgemäßen Beschleunigers aus 1,12-Diamino-dodecan, Acrylsäuremethylester und Hydrazinhydrat

Gemäß EP 0 106 635 - Beispiel 4 - wird das Reaktionsprodukt aus 1,12-Diaminododecan, Acrylsäuremethylester und Hydrazinhydrat kristallin erhalten.

IIa Herstellung einer Prepreg-Reaktionsmischung aus II unter Mitverwendung von Dicyandiamid

101 g Dimethylformamid p. a. werden wegen der mangelnden Löslichkeit des Reaktionsprodukts II in diesem Lösungsmittel zunächst auf 80 °C erhitzt und anschließend mit
3 g Reaktionsprodukt II und
16 g Dicyandiamid
versetzt. Diese Lösung wird mit 60 phr mit einem Epoxidharz (Epoxid-Äquivalentgewicht ca. 190) abgemischt und nach dem Abkühlen auf Raumtemperatur zur Prepreg-Herstellung eingesetzt.

Die Herstellung und Weiterverarbeitung der Prepregs erfolgt in der für Beispiel I a beschriebenen Art und Weise, allerdings ist das Endprodukt bei 120 °C Preßtemperatur auch nach Verdreifachung der Preßzeit von 30 auf 90 min noch derart schwach ausgehärtet, daß eine Ermittlung der Übergangstemperaturen im Torsionsschwingversuch an diesem Produkt nicht möglich ist. Die restlichen Merkmale dieses Beispiels enthält in vergleichender Übersicht ebenfalls Tabelle 1.

II b Herstellung einer Prepreg-Reaktionsmischung aus II unter Mitverwendung von Dicyandiamid

Die entsprechend II a hergestellte Lösung von
16 g Dicyandiamid und
6 g Reaktionsprodukt II in
98 g Dimethylformamid p. a.
wird mit 60 phr mit einem Epoxidharz (Epoxid-Äquivalentgewicht ca. 190) abgemischt und entsprechend Beispiel II a zur Prepreg-Herstellung eingesetzt. Eigenschaften vgl. Tabelle 1).

III Einsatz eines nicht erfindungsgemäßen N-substituierten Imidazolderivates - N-Methylimidazol - als Beschleuniger in Kombination mit Dicyandiamid

III a Herstellung einer Prepreg-Reaktionsmischung aus N-Methylimidazol unter Mitverwendung von Dicyandiamid

Die Lösung von
16 g Dicyandiamid und
3 g N-Methylimidazol in
101 g Dimethyldormamid p. a.
wird mit 60 phr mit einem Epoxidharz (Epoxid-Äquivalentgewicht ca. 190) abgemischt und entsprechend Beispiel I a zur Prepreg-Herstellung eingesetzt.

Eigenschaften vgl. Tabelle 1.

III b Herstellung einer Prepreg-Reaktionsmischung aus N-Methylimidazol unter Mitverwendung von Dicyandiamid

Die Lösung von
16 g Dicyandiamid
6 g N-Methylimidazol in
98 g Dimethylformamid p. a.
wird mit 60 phr mit einem Epoxidharz (Epoxid-Äquivalentgewicht ca. 190) abgemischt und entsprechend Beispiel I a zur Prepreg-Herstellung eingesetzt.

Eigenschaften vgl. Tabelle 1.

IV Herstellung einer Prepreg-Reaktionsmischung aus I ohne Mitverwendung von Dicyandiamid und ohne Lösungsmittel

100 g eines Epoxidharzes (Epoxid-Äquivalentgewicht ca. 190) werden bei Raumtemperatur mit 5 g des erfindungsgemäßen Reaktionsprodukts I vermischt und zur Prepreg-Herstellung eingesetzt. Diese Mischung besitzt bei Raumtemperatur eine Viskosität von 19,5 Pa.s und ist auch nach 10 h noch verarbeitungsfähig.

Die Herstellung der Prepregs im Labormaßstab erfolgt durch Aufstreichen des Reaktionsgemisches auf ein ca. 0,1 $m^2$ großes Glasfilamentgewebe in Atlasbindung, welches nach der Imprägnierung beidseitig mit Trennfolien kaschiert und bei Raumtemperatur gelagert wird.

Nach einer Zwischenlagerung von 24 h bei Raumtemperatur ist das Material soweit gereift, daß es als schwach klebriger Prepreg in mehreren Lagen im Heißpreßverfahren bei 0,1 bar und Temperaturen von 100 - 120 °C in 30 min bis 1 h zu hochfesten Formteilen weiterverarbeitet werden kann. Das derart ausgehärtete Endprodukt zeigt keinerlei Mängel bezüglich der Haftung der einzelnen Prepreglagen und besitzt nach Tabelle 2 eine im Torsionsschwingversuch (DIN 53445) ermittelte Übergangstemperatur von mehr als 170 °C. Die übrigen in Tabelle 2 noch enthaltenen Daten wurden auf die gleiche Weise gemessen, wie in Beispiel I a beschrieben.

Die Prepregs können auch nach 8 Tagen Raumtemperatur entsprechend den vorstehend beschriebenen Verarbeitungsbedingungen noch ohne Eigenschaftsverlust ausgehärtet werden (Tabelle 2), und durch eine Lagerung bei z. B. 6 °C (Kühlschrank) läßt sich dieser Zeitraum auf mehrere Wochen aus dehnen. Weiterhin zeigen die ausgehärteten Materialien auch nach 10 Tagen kombinierter Temperatur (70 °C) und Feuchteeinwirkung (75 % relative Luftfeuchtigkeit) nicht die von den meisten anderen Epoxidsystemen bekannte Verschlechterung der Übergangstemperatur.

V Herstellung einer Prepreg-Reaktionsmischung aus einem nicht erfindungsgemäßen N-substituierten Imidazolderivates - N-Methylimidazol - ohne Mitverwendung von Dicyandiamid und ohne Lösungsmittel

100 g eines Epoxidharzes (Epoxid-Äquivalentgewicht ca. 190) werden bei Raumtemperatur mit 5g N-Methylimidazol vermischt und entsprechend Beispiel IV zur Prepregherstellung eingesetzt.

Eigenschaften vgl. Tabelle 2.

Tabelle 1

| Beispiel | Viskosität der Imprägnierlösung in mPa.s nach | | | DSC-Aufheizrate 10 °C/min | | DSC-Aufheizrate 20 °C/min | | Übergangstemp. lt. DIN 53445 nach Härtung von 30 min bei 120 °C |
|---|---|---|---|---|---|---|---|---|
| | Herst. | 1 Tag | (X) Tagen | Anspringtemp. °C | Peaktemp. °C | Anspringtemp. °C | Peaktemp. °C | °C |
| Ia | 70 | 70 | 90 (8) | 125 | 156 | 137 | 173 | 148 |
| IIa | 70 | 70 | 80 (6) | 135 | 183 | 155 | 203 | nicht ausgehärtet |
| IIIa | 60 | 90 | 130 (2) | 70 | 126 | 105 | 157 | 133 |
| Ib | 80 | 80 | 180 (6) | 111 | 149 | 122 | 165 | 148 |
| IIb | 70 | 80 | 100 (6) | 135 | 183 | 150 | 200 | nicht ausgehärtet* |
| IIIb | 70 | 160 | 520 (2) | 85 | 144 | 95 | 160 | 128 |

*) Tg = 138 °C nach Härtung von 90 min 120 °C + 60 min 150 °C

Tabelle 2

| Beispiel | DSC-Aufheizrate 10 °C/min | | Lagerzeit bei Raumtemperatur in Tagen | Härtungsbedingungen | Übergangstemp. lt. DIN 53 445 | Übergangstemp. nach 10 Tagen Lagerung bei 70 °C u.75% r.F. |
|---|---|---|---|---|---|---|
| | Anspringtemp. °C | Peaktemp. °C | | | °C | °C |
| IV | 70 | 143 | 1 | 30min 90 °C | 171 | |
| | | | 1 | 1 h 120 °C | 174 | |
| | | | 4 | 1 h 120 °C | 173 | 173 |
| | | | 8 | 1 h 120 °C | 173 | |
| V | 65 | 128 | 1 | 30min 90 °C | 151 | |
| | | | 4 | 1 h 120 °C | 150 | 150 |
| | | | 8 | 1 h 120 °C | 150 | |

**Ansprüche**

1. Verbindungen der allgemeinen Formel (I)

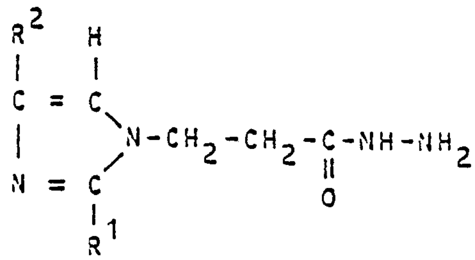

(I)

in welcher $R^1$ und $R^2$ unabhängig voneinander die Bedeutung H, $-CH_3$, $-C_2H_5$, Phenyl haben können.

2. Verwendung von Verbindungen der allgemeinen Formel (I),

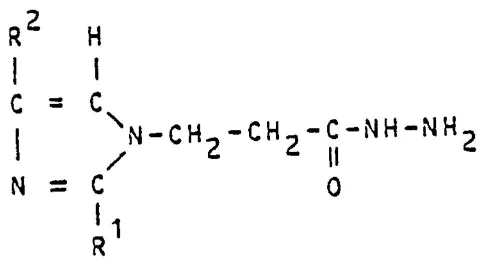

(I)

in welcher $R^1$ und $R^2$ unabhängig voneinander die Bedeutung H, $-CH_3$, $-C_2H_5$, Phenyl haben können, gegebenenfalls unter Mitverwendung üblicher stickstoffhaltiger heterocyclischer Aminverbindungen als Härtungsmittel für Epoxidharze.

3. Härtbare Epoxidharz-Zusammensetzungen enthaltend

a) ein Epoxidharz mit durchschnittlich mehr als einer Epoxidgruppe pro Molekül und gegebenenfalls

b) Dicyandiamid und gegebenenfalls

c) Lösungsmittel, Füllstoffe, Verstärkungs- oder Einlagematerialien, Pigmente, Hilfsstoffe und

d) Verbindungen der allgemeinen Formel (I)

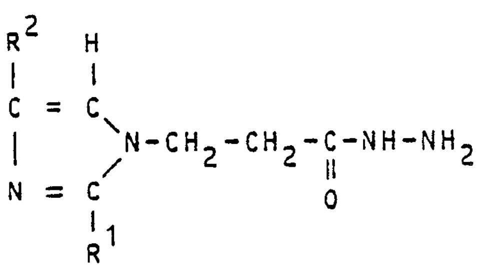

(I)

in welcher $R^1$ und $R^2$ unabhängig voneinander die Bedeutung H, $-CH_3$, $-C_2H_5$, Phenyl haben können und gegebenenfalls

e) übliche stickstoffhaltige heterocyclische Aminverbindungen.

4. Härtbare Epoxidharz-Zusammensetzungen, worin die Verstärkungs- oder Einlagematerialien bei Raumtemperatur mit dem Bindemittel bestehend aus

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

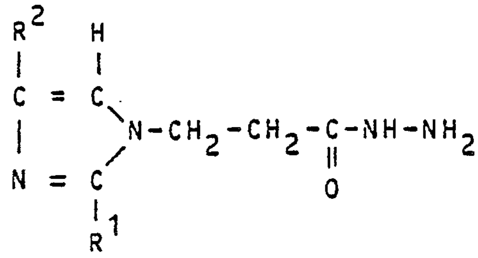

(I)

in welcher $R^1$ und $R^2$ unabhängig voneinander die Bedeutung H, $-CH_3$, $-C_2H_5$, Phenyl haben können und gegebenenfalls

e) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

5. Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

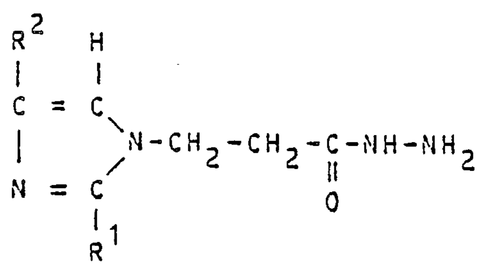

(I)

in welcher $R^1$ und $R^2$ unabhängig voneinander die Bedeutung H, $-CH_3$, $-C_2H_5$, Phenyl haben können und gegebenenfalls

e) üblichen stickstoffhaltigen heterocyclischen Aminverbindungen

getränkt und gegebenenfalls in den halbfesten aber noch schmelzbaren Zustand (B-Zustand) überführt werden und in zweiter Stufe die feuchten Laminate oder Prepregs und unter Formgebung oder zwischen zu verklebende Substrate gebracht, unter Anwendung von Druck bei erhöhter Temperatur ausgehärtet werden.

Patentansprüche für folgenden Vertragsstaat : ES

1. Verfahren zur Herstellung eines Härtungsmittels für Epoxidharze, dadurch gekennzeichnet, daß in erster Stufe zu einer Lösung von 2-Ethyl-4-methyl-imidazol bei erhöhter Temperatur unter intensiver Vermischung Acrylsäureester zugegeben und nach Vervollständigung der Additionsreaktion in zweiter Stufe diese Reaktionsmischung durch Zugabe zu einer vorgewärmten Hydrazinhydratlösung zum Hydrazid umgesetzt wird, wobei pro Mol 2-Ethyl-4-methyl-imidazol, 0,5 Mol Acrylsäureester und 0,5 Mol Hydrazin eingesetzt werden.

2.Verwendung von Verbindungen der allgemeinen Formel (I),

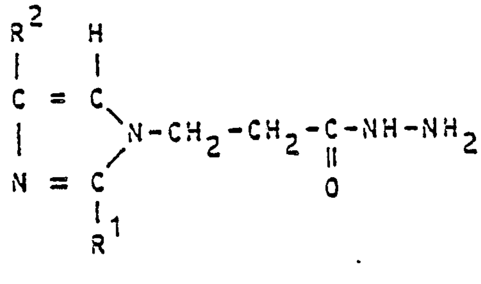

(I)

in welcher $R^1$ und $R^2$ unabhängig voneinander die Bedeutung H, $-CH_3$, $-C_2H_5$, Phenyl haben können,

gegebenenfalls unter Mitverwendung üblicher stickstoffhaltiger heterocyclischer Aminverbindungen als Härtungsmittel für Epoxidharze.

3. Verfahren zur Herstellung härtbarer Epoxidharz-Zusammensetzungen durch Vermischung von

a) ein Epoxidharz mit durchschnittlich mehr als einer Epoxidgruppe pro Molekül und gegebenenfalls

b) Dicyandiamid und gegebenenfalls

c) Lösungsmittel, Füllstoffe, Verstärkungs- oder Einlagematerialien, Pigmente, Hilfsstoffe und

d) Verbindungen der allgemeinen Formel (I)

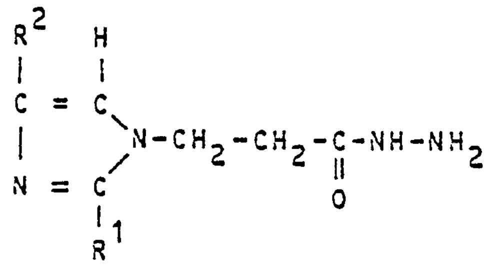

(I)

in welcher $R^1$ und $R^2$ unabhängig voneinander die Bedeutung H, $-CH_3$,.$-C_2H_5$, Phenyl haben können und gegebenenfalls

e) übliche stickstoffhaltige heterocyclische Aminverbindungen.

4. Verfahren zur Herstellung härtbarer Epoxidharz-Zusammen setzungen, worin

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

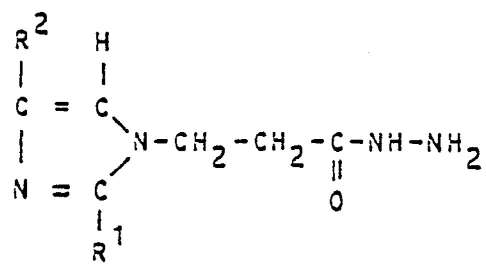

.(I)

in welcher $R^1$ und $R^2$ unabhängig voneinander die Bedeutung H, $-CH_3$, $-C_2H_5$, Phenyl haben können und gegebenenfalls

e) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

5. Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül und gegebenenfalls

b) Dicyandiamid und gegebenenfalls

c) Lösungsmitteln, Füllstoffen, Verstärkungs- oder Einlagematerialien, Pigmenten, Hilfsstoffen und

d) Verbindungen der allgemeinen Formel (I)

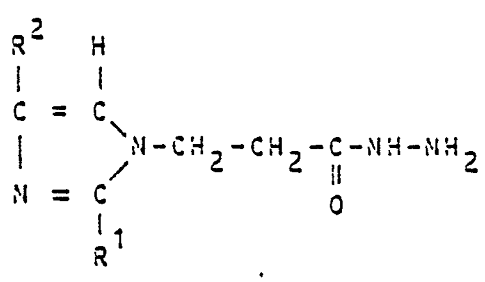

(I)

in welcher $R^1$ und $R^2$ unabhängig voneinander die Bedeutung H, $-CH_3$, $-C_2H_5$, Phenyl haben können und

gegebenenfalls
e) üblichen stickstoffhaltigen heterocyclischen Aminverbindungen
getränkt und gegebenenfalls in den halbfesten aber noch schmelzbaren Zustand (B-Zustand) überführt werden und in zweiter Stufe die feuchten Laminate oder Prepregs und unter Formgebung oder zwischen zu verklebende Substrate gebracht, unter Anwendung von Druck bei erhöhter Temperatur ausgehärtet werden.

Europäisches Patentamt

# EUROPÄISCHER RECHERCHENBERICHT

Nummer der Anmeldung

EP 89 11 0645

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 104 837 (AJINOMOTO)<br>* Ansprüche; Seiten 4-6 *<br>--- | 1-5 | C 07 D 233/54<br>C 08 G 59/40 |
| Y | EP-A-0 024 119 (MOBIL OIL)<br>* Ansprüche *<br>--- | 1-5 | |
| A | JOURNAL OF APPLIED POLYMER SCIENCE, Band 13, 1969, Seiten 227-232; D. AELONY: "Storage-stable epoxy B-stage resins"<br>* Seiten 227-232 *<br>--- | 1-2 | |
| A | CHEMICAL ABSTRACTS, Band 82, Nr. 25, 23. Juni 1975, Zusammenfassung Nr. 170942u, Columbus, Ohio, US; & JP-A-74 41 434 (SHIKOKU-KASEI KOGYO CO. LTD) 08-11-1974<br>* Zusammenfassung *<br>----- | 1-2 | |
| | | | RECHERCHIERTE SACHGEBIETE (Int. Cl.5) |
| | | | C 07 D<br>C 08 G |

Der vorliegende Recherchenbericht wurde für alle Patentansprüche erstellt

| Recherchenort | Abschlußdatum der Recherche | Prüfer |
|---|---|---|
| DEN HAAG | 23-10-1989 | DERAEDT G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)